(19) Europäisches Patentamt
European Patent Office
Office européen des brevets

(11) Publication number : 0 442 204 A2

# (12) EUROPEAN PATENT APPLICATION

(21) Application number : 90313384.1

(22) Date of filing : 10.12.90

(51) Int. Cl.[5] : C07D 487/04, A61K 31/505, // C07D239/48, C07D239/56, (C07D487/04, 239:00, 239:00)

The title of the invention has been amended (Guidelines for Examination in the EPO, A-III, 7.3).

(30) Priority : 15.12.89 GB 8928346

(43) Date of publication of application : 21.08.91 Bulletin 91/34

(84) Designated Contracting States : CH DE FR GB IT LI NL

(71) Applicant : SMITH KLINE & FRENCH LABORATORIES LIMITED
Mundells
Welwyn Garden City Hertfordshire, AL7 1EY (GB)

(72) Inventor : Coates, William John
Smithkline Beecham Pharmaceuticals, The Frythe
Welwyn, Hertfordshire AL6 9AR (GB)

(74) Representative : Thompson, Clive Beresford et al
SmithKline Beecham plc Corporate Patents
Mundells
Welwyn Garden City, Herts AL7 2EY (GB)

## (54) Pyrimidopyrimidine derivatives, process and intermediates for their preparation and pharmaceutical compositions containing them.

(57) Compounds of the formula (1) :

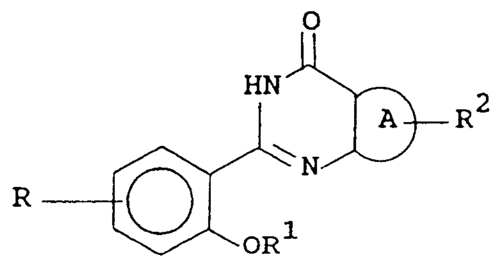

(1)

and pharmaceutically acceptable salts are described wherein $R^1$ is $C_{1-6}$alkyl, $C_{2-6}$alkenyl, $C_{3-5}$cycloalkyl $C_{1-6}$alkyl, or $C_{1-6}$alkyl substituted by 1 to 6 fluoro groups ; $R^2$ is $C_{1-6}$alkylthio, $C_{1-6}$alkylsulphonyl, $C_{1-6}$alkoxy, hydroxy, hydrogen, hydrazino, $C_{1-6}$alkyl, phenyl, $-NHCOR^3$ wherein $R^3$ is hydrogen or $C_{1-6}$alkyl, or $-NR^4R^5$, wherein $R^4$ and $R^5$ together with the nitrogen atom to which they are attached form a pyrrolidino, piperidino, hexahydroazepino, morpholino or piperazino ring, or $R^4$ and $R^5$ are independently hydrogen, $C_{3-5}$cycloalkyl or $C_{1-6}$alkyl which is optionally substituted by $-CF_3$, phenyl, $-S(O)_nC_{1-6}$alkyl wherein n is 0, 1 or 2, $-OR^6$, $-CO_2R^7$ or $-NR^8R^9$ wherein $R^6$ to $R^9$ are independently hydrogen or $C_{1-6}$alkyl, provided that the carbon atom adjacent to the nitrogen atom is not substituted by said $-S(O)_nC_{1-6}$alkyl, $-OR^6$ or $-NR^8R^9$ groups ; R is halo, $C_{1-4}$alkyl, $C_{1-4}$ alkoxy, cyano, $-CONR^{10}R^{11}$, $CO_2R^{12}$, $C_{1-4}$alkylS(O)$_n$, $-NO_2$, $-NH_2$, $-NHCOR^{13}$ or $SO_2NR^{14}R^{15}$ wherein n is 0, 1 or 2 and $R^{10}$ to $R^{15}$ are independently hydrogen

or $C_{1-4}$alkyl; and 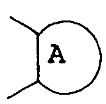 is a ring of sub-formula (a) or (b) :

EP 0 442 204 A2

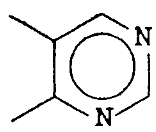

(a)

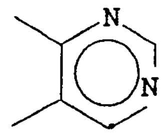

(b).

These compounds have bronchodilatator, anti-allergic, and vasodilator activities. Pharmaceutical compositions are described as are methods of use. Processes for the preparation of compounds of the formula (1) are described.

## CHEMICAL COMPOUNDS

The present invention relates to pyrimidopyrimidine derivatives, processes for their preparation, intermediates in their preparation, their use as therapeutic agents and to pharmaceutical compositions containing them. The compounds of this invention are inhibitors of a calmodulin insensitive cyclic GMP phosphodiesterase and are of use in combatting such conditions where such inhibition is thought to be beneficial. They are bronchodilators and are therefore of use in combatting chronic reversible obstructive lung diseases such as asthma and bronchitis. Some of the compounds of the present invention have anti-allergic activity and are therefore useful in combatting allergic diseases such as allergic asthma, allergic rhinitis, urticaria and gastrointestinal motility disorders such as irritable bowel syndrome. Furthermore the compounds of this invention are vasodilators and are therefore of value in combatting angina, hypertension and congestive heart failure.

Accordingly the present invention provides compounds of the formula (1) :

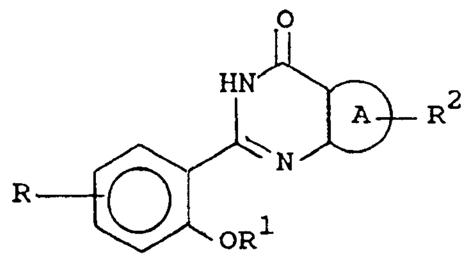

(1)

and pharmaceutically acceptable salts thereof, wherein

$R^1$ is $C_{1-6}$alkyl, $C_{2-6}$alkenyl, $C_{3-5}$cycloalkyl $C_{1-6}$alkyl, or $C_{1-6}$ alkyl substituted by 1 to 6 fluoro groups ;

$R^2$ is $C_{1-6}$alkylthio, $C_{1-6}$alkylsulphonyl, $C_{1-6}$alkoxy, hydroxy, hydrogen, hydrazino, $C_{1-6}$alkyl, phenyl, -$NHCOR^3$ wherein $R^3$ is hydrogen or $C_{1-6}$ alkyl, or -$NR^4R^5$, wherein $R^4$ and $R^5$ together with the nitrogen atom to which they are attached form a pyrrolidino, piperidino, hexahydroazepino, morpholino or piperazino ring, or $R^4$ and $R^5$ are independently hydrogen, $C_{3-5}$cycloalkyl or $C_{1-6}$alkyl which is optionally substituted by -$CF_3$, phenyl, -$S(O)_nC_{1-6}$alkyl wherein n is 0, 1 or 2, -$OR^6$, -$CO_2R^7$ or -$NR^8R^9$ wherein $R^6$ to $R^9$ are independently hydrogen or $C_{1-6}$alkyl, provided that the carbon atom adjacent to the nitrogen atom is not substituted by said -$S(O)_nC_{1-6}$alkyl, -$OR^6$ or -$NR^8R^9$ groups ;

R is halo, $C_{1-4}$alkyl, $C_{1-4}$alkoxy, cyano, -$CONR^{10}R^{11}$,$CO_2R^{12}$, $C_{1-4}$ alkyl$S(O)_n$, -$NO_2$, -$NH_2$, -$NHCOR^{13}$ or $SO_2NR^{14}R^{15}$ wherein n is 0, 1 or 2 and $R^{10}$ to $R^{15}$ are independently hydrogen or $C_{1-4}$alkyl ; and

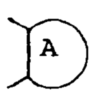 is a ring of sub-formula (a) or (b) :

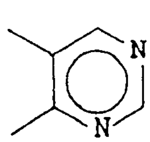

(a)

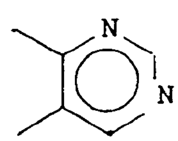

(b).

Suitably $R^1$ is $C_{2-5}$alkyl for example ethyl, n-propyl, isopropyl, butyl, isobutyl or pentyl.

Suitably $R^1$ is $C_{3-5}$alkenyl for example propenyl, butenyl or pentenyl.

Suitably $R^1$ is cyclopropylmethyl.

Examples of $C_{1-6}$alkyl substituted by 1 to 6 fluoro groups include -$CF_3$, -$CH_2CF_3$ or -$CF_2CHFCF_3$.

Preferably $R^1$ is n-propyl.

Suitably $R^2$ is $C_{1-6}$alkylthio, $C_{1-6}$alkylsulphonyl or $C_{1-6}$alkoxy for example methylthio, ethylthio, methylsulphonyl, ethylsulphonyl, methoxy, ethoxy or propoxy.

Suitably $R^2$ is hydroxy, hydrogen or hydrazino.

Suitably $R^2$ is phenyl or $C_{1-6}$alkyl for example methyl, ethyl or propyl.

Suitably $R^2$ is $-NHCOR^3$ for example formamido or acetamido.

Suitably $R^2$ is $-NR^4R^5$ for example amino, methylamino, ethylamino, propylamino, dimethylamino, diethylamino, dipropylamino, cyclopropylamino, morpholino, 2,2,2-trifluoroethylamino, phenethylamino, 3-methylthiopropylamino, 3-methylsulphinylpropylamino, 3-methylsulphonylpropylamino, 2-hydroxyethylamino, 3-hydroxypropylamino, 2-hydroxypropylamino, 3-methoxypropylamino, N-ethyl-N-(2-hydroxyethyl)amino, 2-aminoethylamino, 2-dimethylaminoethylamino, ethoxycarbonylmethylamino, carboxymethylamino, 2-ethoxycarbonylethylamino or 2-carboxyethylamino.

Suitably R is halo for example fluoro, chloro, bromo or iodo.

Suitably R is $C_{1-4}$alkyl or $C_{1-4}$alkoxy for example methyl, ethyl, methoxy, ethoxy or propoxy.

Suitably R is cyano, $-CONR^{10}R^{11}$ for example $-CONH_2$, $-CONHCH_3$ or $-CON(CH_3)_2$ or $-CO_2R^{12}$ wherein $R^{12}$ is $C_{1-4}$alkyl.

Suitably R is $-NO_2$, $-NH_2$ or $-NHCOR^{13}$ wherein $R^{13}$ is $C_{1-4}$alkyl.

Suitably R is $C_{1-4}$alkylS(O)$_n$ for example methylthio, ethylthio, methylsulphinyl, ethylsulphinyl, methylsulphonyl or ethylsulphonyl.

Suitably R is $SO_2NR^{14}R^{15}$ for example sulphamoyl, N-methylsulphamoyl or N,N-dimethylsulphamoyl.

Suitably 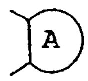 is a group of sub-formula (a) thus

forming a pyrimido[4,5-d]pyrimidine ring system.

Suitably 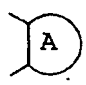 is a group of sub-formula (b) thus

forming a pyrimido[5,4-d]pyrimidine ring system.

Particular compounds of this invention are :

2-(5-cyano-2-propoxyphenyl)-7-methylthiopyrimido-[4,5-d]]-pyrimidin-4(3H)-one,

2-(5-carboxamido-2-propoxyphenyl)-7-methylthiopyrimido-[4,5-d]pyrimido-4(3H)-one, or

2-(5-carboxamido-2-propoxyphenyl)-7-cyclopropylamino-[4,5-d]pyrimido-4(3H)-one,

or pharmaceutically acceptable salts thereof.

This invention covers all tautomeric and optical isomeric forms of compounds of formula (1).

Compounds of the formula (1) wherein $R^2$ is $-NR^4R^5$ or hydrazino or R is $-NH_2$ may form pharmaceutically acceptable salts with acids such as hydrochloric, hydrobromic, sulphuric and phosphoric acids.

Compounds of the formula (1) may form pharmaceutically acceptable salts with metal ions, such as alkali metals for example sodium and potassium, or with an ammonium ion.

In order to use a compound of the formula (1) or a pharmaceutically acceptable salt thereof for the treatment of humans and other mammals it is normally formulated in accordance with standard pharmaceutical practice as a pharmaceutical composition.

Compounds of formula (1) and their pharmaceutically acceptable salts may be administered in standard manner for the treatment of the indicated diseases, for example orally, parenterally, transdermally, rectally, via inhalation or via buccal administration.

Compounds of formula (1) and their pharmaceutically acceptable salts which are active when given orally or via buccal administration can be formulated as liquids, syrups, tablets, capsules and lozenges. An oral liquid formulation will generally consist of a suspension or solution of the compound or salt in a liquid carrier for example, ethanol, glycerine or water with a flavouring or colouring agent. Where the composition is in the form of a tablet, any pharmaceutical carrier routinely used for preparing solid formulations may be used. Examples of such carriers include starch, celluloses, lactose, sucrose and magnesium stearate. Where the composition is in the form of a capsule, any routine encapsulation is suitable, for example using the aforementioned carriers in a hard gelatin capsule shell. Where the composition is in the form of a soft gelatin shell capsule any pharmaceutical carrier routinely used for preparing dispersions or suspensions may be considered, for example aqueous gums, celluloses, silicates or oils and are incorporated in a soft gelatin capsule shell.

Typical parenteral compositions consist of a solution or suspension of the compound or salt in a sterile aqueous or non-aqueous carrier optionally containing a parenterally acceptable oil, for example polyethylene glycol, polyvinyl-pyrrolidone, lecithin, arachis oil, or sesame oil.

A typical suppository formulation comprises a compound of formula (1) or a pharmaceutically acceptable salt thereof which is active when administered in this way, with a binding and/or lubricating agent, for example

polymeric glycols, gelatins, cocoa-butter or other low melting vegetable waxes or fats.

Typical transdermal formulations comprise a conventional aqueous or non-aqueous vehicle, for example a cream, ointment, lotion or paste or are in the form of a medicated plaster, patch or membrane.

Typical compositions for inhalation are in the form of a solution, suspension or emulsion that may be administered in the form of an aerosol using a conventional propellant such as dichlorodifluoromethane or trichlorofluoromethane, or are in the form of a powder for insufflation.

Preferably the composition is in unit dosage form, for example a tablet, capsule or metered aerosol dose, so that the patient may administer to himself a single dose.

Each dosage unit for oral administration contains suitably from 0.001 mg/Kg to 30 mg/Kg, and preferably from 0.005 mg/Kg to 15 mg/Kg, and each dosage unit for parenteral administration contains suitably from 0.001 mg/Kg to 10 mg/Kg, of a compound of formula (1) or a pharmaceutically acceptable salt thereof calculated as the free base.

The daily dosage regimen for oral administration is suitably about 0.001 mg/Kg to 120 mg/Kg, of a compound of formula (1) or a pharmaceutically acceptable salt thereof calculated as the free base. The daily dosage regimen for parenteral administration is suitably about 0.001 mg/Kg to 40 mg/Kg, for example about 0.005 mg/Kg to 10 mg/Kg, of a compound of the formula (1) or a pharmaceutically acceptable salt thereof calculated as the free base. The active ingredient may be administered as required for example from 1 to 8 times a day or by infusion. The compositions of the invention are bronchodilators and are useful in chronic reversible obstructive lung disease for example asthma and bronchitis. In addition some of the compositions of the present invention have anti-allergic activity and are useful in combatting allergic diseases such as allergic asthma, allergic rhinitis, urticaria and irritable bowel syndrome. The compositions of the present invention also have vasodilator activity and are of use in the treatment of angina, hypertension and congestive heart failure. Such conditions can be treated by administration orally, topically, rectally, parenterally or by inhalation. For administration by inhalation dosages are controlled by a valve, are administered as required and for an adult are conveniently in the range 0.1-5.0 mg of a compound of the formula (1) or a pharmaceutically acceptable salt thereof.

The compounds of this invention may be co-administered with other pharmaceutically active compounds, for example in combination, concurrently or sequentially. Conveniently the compounds of this invention and the other active compound or compounds are formulated in a pharmaceutical composition. Examples of compounds which may be included in pharmaceutical compositions with the compounds of the formula (1) are bronchodilators such as sympathomimetic amines for example isoprenaline, isoetharine, sulbutamol, phenylephrine and ephedrine or xanthine derivatives for example theophylline and aminophylline, anti-allergic agents for example disodium cromoglycate, histamine $H_1$-antagonists, vasodilators for example hydralazine, angiotensin converting enzyme inhibitors for example captopril, anti-anginal agents for example isosorbide nitrate, glyceryl trinitrate and pentaerythritol tetranitrate, anti-arrhythmic agents for example quinidine, procainamide and lignocaine, calcium antagonists for example verapamil and nifedipine, diuretics such as thiazides and related compounds for example bendrofluazide, chlorothiazide, chlorothalidone, hydrochlorothiazide, and other diuretics for example frusemide and triamterene, and sedatives for example nitrazepam, flurazepam and diazepam.

In another aspect the present invention provides a process for the preparation of compounds of the formula (1) or pharmaceutically acceptable salts thereof, which process comprises :

a) cyclising a compound of the formula (2) :

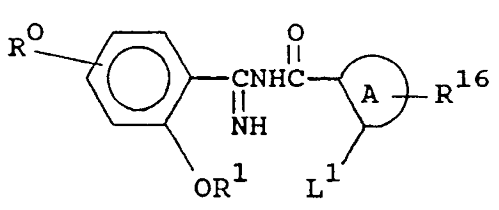

(2)

wherein $L^1$ is a displaceable group, $R^1$ and 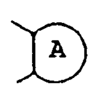 are as hereinbefore defined, $R^{16}$ is a group $R^2$ as hereinbefore defined or a precursor thereof and $R^0$ is a group R as hereinbefore defined or a precursor thereof ; or

b) cyclising a compound of the formula (3) :

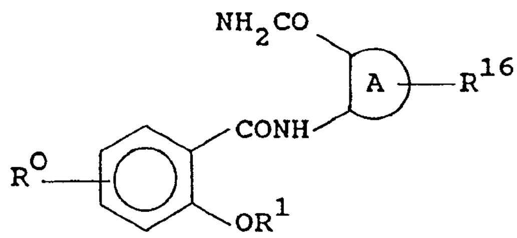

(3)

wherein $R^1$, $R^O$, $R^{16}$ and 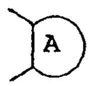 are as hereinbefore defined;

and thereafter where necessary :

. converting a group $R^{16}$ to a group $R^2$ ;

. converting a group $R^0$ to a group R ;

. optionally forming a pharmaceutically acceptable salt.

Suitably the cyclisation of a compound of the formula (2) is performed in the presence of a base such as an alkali metal carbonate or triethylamine, in an aprotic solvent such as dimethylformamide, acetonitrile or N-methylpyrrolidone, at ambient or an elevated temperature, for example 50-170°C, conveniently at the reflux temperature of the reaction mixture. Suitably $L^1$ is halo for example bromo or chloro.

Suitably a compound of the formula (3) is cyclised by heating at an elevated temperature, for example 50-150°C, in the presence of an acid or a base in a suitable solvent such as aqueous $C_{1-4}$alcohols, water, toluene, a halohydrocarbon or acetonitrile. Conveniently a compound of the formula (3) is cyclised by heating in pyridine or aqueous base such as sodium hydroxide at the reflux temperature of the reaction mixture.

Examples of $R^{16}$ being a precursor to a group $R^2$ is when $R^{16}$ is a halo or $C_{1-6}$alkylthio group. Such groups can be converted to a $-NR^4R^5$ group by reaction with an amine $HNR^4R^5$ in a suitable solvent such as a $C_{1-4}$-alkanol or pyridine at an elevated temperature, for example 50-120°C, conveniently in a pressure vessel.

A compound of the formula (1) wherein $R^2$ is $C_{1-6}$alkylthio can suitably be converted to the corresponding compound wherein $R^2$ is $C_{1-6}$alkylsulphonyl by reaction with an oxidising agent, for example with at least two equivalents of a peroxy acid such as m-chloroperoxybenzoic acid.

A compound of the formula (1) wherein $R^2$ is $C_{1-6}$alkylsulphonyl can suitably be converted to the corresponding compound wherein $R^2$ is $-NR^4R^5$ by reaction with an amine $HNR^4R^5$ in a suitable solvent such as a halohydrocarbon or toluene at ambient or elevated temperature for example 40-100°C.

A compound of the formula (1) wherein $R^2$ is $C_{1-6}$alkylsulphonyl can suitably be converted to the corresponding compound wherein $R^2$ is $C_{1-6}$alkoxy by reaction with a $C_{1-6}$alkoxide, eg an alkali metal $C_{1-6}$alkoxide such as sodium methoxide or ethoxide, in a $C_{1-6}$alkanol at ambient or elevated temperature, for example 40-100°C.

A compound of the formula (1) wherein $R^2$ is $C_{1-6}$alkylthio can suitably be converted to the corresponding compound wherein $R^2$ is hydrazino by reaction with hydrazine.

A compound of the formula (1) wherein $R^2$ is hydrazino can be converted to the corresponding compound wherein $R^2$ is hydrogen by treatment with silver oxide.

A compound of the formula (1) wherein $R^2$ is hydroxy can suitably be prepared by reacting a compound of the formula (1) wherein $R^2$ is $C_{1-6}$alkylthio with an alkali metal $C_{1-6}$alkoxide such as sodium methoxide or ethoxide under aqueous work-up conditions.

A compound of the formula (1) wherein $R^2$ is $C_{1-6}$alkoxy can be converted to the corresponding compound wherein $R^2$ is hydroxy by hydrolysis, for example by treatment with hydrochloric acid.

A compound of the formula (1) wherein $R^2$ is amino can suitably be converted to the corresponding compound where $R^2$ is $-NHCOR^3$ by reaction with a formylating or $C_{2-7}$alkanoylating agent. Examples of such reagents include formic acid, $C_{1-6}$alkyl formate, formamide, acetic anhydride, propionic anhydride or acetylchloride.

A compound of the formula (1) wherein $R^4$ or $R^5$ is $C_{1-6}$alkyl substituted by $C_{1-6}$alkylthio can suitably be converted to the corresponding compound wherein $R^4$ or $R^5$ is $C_{1-6}$alkyl substituted by $C_{1-6}$alkylsulphinyl by reaction with one equivalent of an oxidising agent such as a peroxy acid, for example m-chloroperoxybenzoic acid. The $C_{1-6}$alkylsulphinyl compound can similarly be oxidised to a compound of the formula (1) wherein $R^4$ or $R^5$ is $C_{1-6}$alkyl substituted by $C_{1-6}$alkylsulphonyl.

A compound of the formula (1) wherein $R^4$ or $R^5$ is $C_{1-6}$alkyl substituted by $-CO_2R^7$ in which $R^7$ is $C_{1-6}$alkyl can suitably be hydrolysed by reaction with aqueous base, for example aqueous sodium hydroxide to form the corresponding compound wherein $R^7$ is hydrogen.

Examples of $R^0$ being a precursor to a group R is when $R^0$ is a $C_{1-4}$alkylthio or $C_{1-4}$alkoxycarbonyl group. Suitably a $C_{1-4}$alkylthio group can be converted to a $C_{1-4}$alkylsulphinyl group by treatment with one mole equivalent of an oxidising agent such as hydrogen peroxide or potassium periodate. A further mole equivalent of an oxidising agent such as hydrogen peroxide or potassium permanganate can be used to convert a $C_{1-4}$alkylsulphinyl group to a $C_{1-4}$alkylsulphonyl group.

A $C_{1-4}$alkoxycarbonyl group can be converted to a $CONR^{10}R^{11}$ group by reaction with an amine $HNR^{10}R^{11}$ wherein $R^{10}$ and $R^{11}$ are as hereinbefore defined.

A compound of the formula (1) wherein R is hydrogen can be converted to the corresponding compound wherein R is 5-nitro by reaction with a suitable nitrating agent, such as fuming nitric acid together with sulphuric acid.

A compound of the formula (1) wherein R is nitro can be converted to the corresponding compound wherein R is amino by reaction with a reducing agent, for example via catalytic hydrogenation with palladium on carbon. If desired the amino group can be converted to a $C_{1-4}$alkanoyl-amino group by treatment with a $C_{1-4}$alkanoylating agent, for example acetic anhydride.

Alternatively a compound of the formula (1) wherein R is amino can be treated with sodium nitrite and an inorganic acid such as sulphuric acid to form a diazonium salt which can be converted to compounds of the formula (1) wherein R is cyano or halo by reaction with cuprous cyanide or cuprous halide.

A compound of the formula (1) wherein R is cyano can be hydrolysed to the corresponding compound wherein R is carboxamido by treatment with concentrated sulphuric acid or by treatment with hydrogen peroxide and potassium hydroxide or by treatment with manganese dioxide on silica.

A compound of the formula (1) wherein R is hydrogen can be converted to the corresponding compound wherein R is $5-SO_2NR^{14}R^{15}$ by reaction with chlorosulphonic acid and thereafter with an amine $HNR^{14}R^{15}$ wherein $R^{14}$ and $R^{15}$ are as hereinbefore defined.

As will be readily understood by the man skilled in the art, reactive groups in other parts of the molecule can be protected before the groups R and $R^2$ are converted as hereinbefore described.

The compounds of the formula (2) can be prepared by reaction of a compound of the formula (4) :

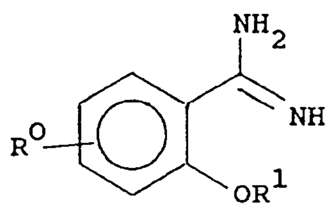

(4)

wherein $R^0$ and $R^1$ are as hereinbefore defined,
with a compound of the formula (5) :

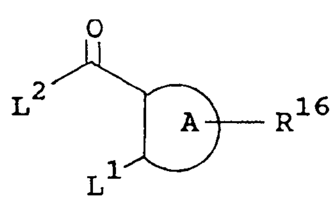

(5)

wherein $L^2$ is a leaving group and $L^1$, $R^{16}$ and 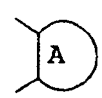 are as hereinbefore defined.

Suitably $L^2$ is $C_{1-6}$alkoxy or halo for example methoxy, ethoxy, chloro or bromo. Conveniently a solution of a compound of the formula (4) is initially formed by treatment of an acid addition salt of a compound of the formula (4) with a suitable base, for example triethylamine, a sodium alkoxide or sodium hydride, in an organic

solvent such as a $C_{1-4}$alkanol, acetonitrile or dimethylformamide and the solution is then treated with a compound of the formula (5) at a moderate temperature for example 0-60°C, conveniently ambient, to afford a compound of formula (2). Suitable acid addition salts are those formed with inorganic acids such as hydrochloric or sulphuric acid or with strong organic acids such as methanesulphonic or p-toluenesulphonic acid. Suitably a compound of the formula (2) is isolated and is then cyclised as hereinbefore described. Alternatively, a compound of the formula (2) is not isolated but is cyclised in situ by stirring at ambient or an elevated temperature, for example 40-170°C.

A compound of the formula (3) can be prepared by reaction of a compound of the formula (6) :

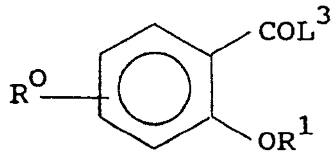

(6)

wherein $R^0$ and $R^1$ are as hereinbefore defined and $L^3$ is halo,
with a compound of the formula (7) :

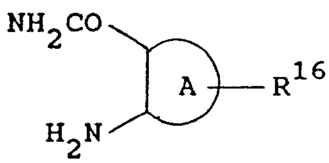

(7)

wherein $R^{16}$ and 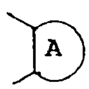 are as hereinbefore defined.

Suitably $L^3$ is chloro or bromo. Suitably a compound of the formula (6) is reacted with a compound of the formula (7) at ambient or elevated temperature e.g. 50-100°C in a suitable solvent such as toluene, acetonitrile or a halohydrocarbon e.g. chloroform or dichloromethane, optionally in the presence of a base such as pyridine or triethylamine, to form a compound of the formula (3) which may be cyclised in situ or may be isolated and thereafter cyclised as hereinbefore described.

The compounds of the formula (4) and acid addition salts thereof are known or preparable in conventional manner from US Patent 3819631.

The compound of the formula (5) are known or can be prepared by methods known in the art.

For example a compound of the formula (5) wherein 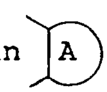 is a group of sub-formula (a), $L^1$ is halo, $L^2$ is $C_{1-6}$-alkoxy and $R^{16}$ is in the 2-position of the pyrimidine ring can be prepared by reaction of a compound of the formula (8) :

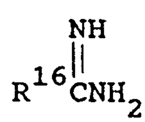

(8)

wherein $R^{16}$ is as hereinbefore defined,
with a compound of the formula (9) :

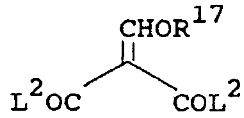 (9)

wherein $R^{17}$ is $C_{1-6}$alkyl and $L^2$ is as hereinbefore defined, and thereafter by reaction with a halogenating agent.

Suitable halogenating agents include thionyl chloride, phosphorous oxychloride or phosphorous tribromide.

A compound of the formula (5) wherein 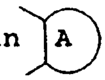 is a group of sub-formula (a), $L^1$ and $L^2$ are chloro and $R^{16}$ is 2-chloro can be prepared by reacting 2,4-dihydroxypyrimidine-5-carboxylic acid with phosphorous oxychloride.

A compound of the formula (5) wherein 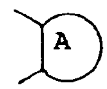 is a group of sub-formula (a), $L^1$ is chloro, $L^2$ is ethoxy and $R^{16}$ is 2-methylthio is commercially available (Aldrich Chemical Co Ltd).

A compound of the formula (5) wherein 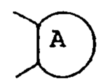 is a group of sub-formula (b), $L^1$ is bromo, $L^2$ is chloro and $R^{16}$ is in the 2-position of the pyrimidine ring can be prepared by reaction of a compound of the formula (8) as hereinbefore defined with mucobromic acid and thereafter by reaction with a chlorinating agent such as thionyl chloride.

The compound of the formula (7) is known or can be prepared by methods known in the art.

For example a compound of the formula (7) wherein

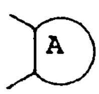 is a group of sub-formula (a) and $R^{16}$ is in the 2-position of the pyrimidine ring can be prepared by hydrolysing a compound of the formula (10) :

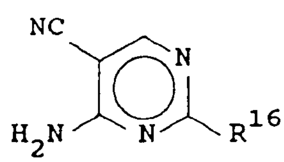 (10)

wherein $R^{16}$ is as hereinbefore defined.

Suitably a compound of the formula (10) is hydrolysed by treatment with concentrated sulphuric acid or by treatment with hydrogen peroxide and potassium hydroxide.

A compound of the formula (10) can be prepared by reacting a compound of the formula (8) as hereinbefore defined with a compound of the formula (11) :

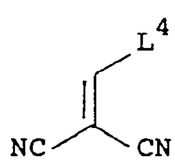 (11)

wherein $L^4$ is a leaving group.

Examples of $L^4$ include $C_{1-6}$alkoxy, $C_{1-6}$alkylthio or halo such as chloro or bromo. Suitably $L^4$ is $C_{1-6}$alkoxy. Alternatively a compound of the formula (7) wherein

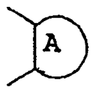 is a group of sub-formula (a) and $R^{16}$ is hydrogen

can be prepared by reacting a compound of the formula (8) wherein $R^{16}$ is hydrogen with cyanoacetamide (Chem. Ber. 98, 3883 (1965)).

Pharmaceutically acceptable acid addition salts of the compounds of the formula (1) wherein $R^2$ is $-NR^4R^5$ or hydrazino or R is $-NH_2$ may be prepared from the corresponding base of the compounds of the formula (1) in conventional manner. For example the base may be reacted with an acid in a $C_{1-4}$alkanol, or an ion-exchange resin may be used. The salts of the compounds of the formula (1) may be interconverted using ion-exchange resins. Non-pharmaceutically acceptable salts are therefore of use as they can be converted to pharmaceutically acceptable salts.

Pharmaceutically acceptable base addition salts of the compounds of the formula (1) may be prepared by standard methods, for example by reacting a solution of the compound of the formula (1) with a solution of the base.

The following biological test methods, data and Examples serve to illustrate this invention.

**Bronchodilatation - In vivo**

Male guinea-pigs of the Dunkin Hartley strain (500-600 g) are anaesthetised with Sagatal (pentobarbital sodium) (60 mg/kg). Airway resistance is measured using a modification of the classical Konzett-Rossler technique (J. Pharm. Methods, 13, 309-315, 1985). U46619 (9,11-methaneoepoxy-$PGH_2$) is infused i.v. at a rate of 2.5 nmol/min, this produced a steady state of bronchoconstriction (approximately 120% increase from basal airway resistance). The compound under test is administered by i.v. bolus injection, and the subsequent peak inhibition of bronchoconstriction is recorded.

The dose of compound required to reduce the U46619-induced bronchoconstriction by 50% is given as the $BD_{50}$.

Anti-allergic activity

Male Duncan Hartley guinea-pigs (250-300 g) are sensitised to ovalbumen by i.p. injection of 2 ml of 50 $mg.ml^{-1}$ i.p. and 0.2 ml s.c. Three weeks later they are anaesthetised with 60 $mg.kg^{-1}$ sodium pentabarbitone. The trachea is cannulated and the animal respires at a rate of 40 breaths per minute and at an initial tracheal inflation pressure of 16 mmHg. Tracheal inflation pressure is measured by a transducer connected to a side arm of the respiration circuit. The carotid artery is cannulated for the measurement of blood pressure and the signal is used to trigger an instantaneous rate meter. A jugular vein is cannulated for the administration of drug and allergen. After surgery the animals are allowed to stabilise and the drug is administered i.v. as a bolus. Following this, ovalbumen 1 $mg.kg^{-1}$ is injected i.v. as the antigen challenge either 2, 15 or 30 minutes following drug treatment and the peak bronchoconstrictor response recorded. For the control group ovalbumen only is given. One ovalbumen challenge per guinea-pig is used and n = 6 for each time point. The percentage increase in tracheal inflation pressure is calculated.

**Phosphodiesterase activity**

The activity of the compounds of the present invention as inhibitors of a calmodulin insensitive cyclic GMP phosphodiesterase is measured using the procedure described in European Patent Application No. 293063.

EXAMPLE 1

Preparation of 2-(5-cyano-2-propoxyphenyl)-7-methylthiopyrimido-[4,5-d]]pyrimidin-4(3H)-one

a. 2-Chloro-5-nitrobenzoic acid (15.11 g) was added, under nitrogen, to sodium propoxide (from 50% sodium hydride in oil, 7.68 g, and propanol, 6.7 ml) in dimethylformamide (100 ml) cooled in a water bath to maintain the temperature below 45°C. After 30 minutes in the cold, the mixture was stirred for 1 hour at room temperature then poured onto crushed ice (500 g) and the mixture acidified with 2N hydrochloric acid

to give 5-nitro-2-propoxybenzoic acid, 15.77 g, m.p. 125.5-127°C.

b. The above acid (15.75 g) in ethanol (250 ml) was hydrogenated at 50 psi in the presence of 10% palladium on charcoal (0.75 g) until the uptake of hydrogen was complete. The mixture was heated with 50% aqueous ethanol (200 ml) and filtered. Evaporation of the filtrate to about 150 ml gave 5-amino-2-propoxybenzoic acid, 7.83 g, m.p. 146-148°C.

c. Sodium nitrite (2.76 g) in water (20 ml) was added to a solution of the above amine (7.8 g) in water (30 ml) containing sodium hydroxide (1.6 g). The solution was cooled to 0°C and slowly added to a mixture of hydrochloric acid (14 ml) and crushed ice (60 g) at -5 to +2°C. After the addition the mixture was stirred at 0°C and excess of nitrous acid was destroyed by the addition of urea, then sodium hydroxide (13.6 g) in water (25 ml) was added below 2°C. The mixture was then added in portions to a vigorously stirred solution at 0°C prepared from water (75 ml), sodium cyanide (5.81 g), cuprous cyanide (3.93 g), and sodium carbonate (4.24 g). The resultant mixture was stirred in the cold for 30 minutes, then at room temperature for 1 hour. In the morning the mixture was treated with 6N hydrochloric acid to pH 2 and extracted with dichloromethane (300 ml). Evaporation of the extract yielded 5-cyano-2-propoxybenzoic acid, 5.02 g, m.p. 118-120°C.

d. A mixture of the above acid (11.1 g) and thionyl chloride (24 ml) was heated under reflux for 2 hours. The residue left after evaporation was dissolved in ether (100 ml) and ammonia gas was passed through the solution until reaction was complete. After addition of water (75 ml) the mixture was extracted with chloroform (200 ml) and the extract was evaporated. Trituration of the residue with ether-petroleum ether (b.p. 40-60°C) gave 5-cyano-2-propoxybenzamide, 9.59 g. Recrystallisation from benzene-acetonitrile gave pure material, m.p. 138-140°C.

e. A solution of the above benzamide (9.35 g) in dichloromethane (100 ml) was treated with a 1 molar solution of triethyloxonium tetrafluoroborate in dichloromethane (55 ml). After 20 hours further Meerwein's reagent (20 ml) was added and the mixture was stirred for a further 24 hours. Evaporation gave the crude imidate which was dissolved in saturated ethanolic ammonia (120 ml) and the solution was stirred overnight at room temperature. After evaporation the residue was partitioned between 1 Normal hydrochloric acid (100 ml) and dichloromethane (100 ml). The organic layer contained unreacted benzamide (4.78 g) which could be recycled. The acidic layer was basified with 6 Normal sodium hydroxide, extracted with ethyl acetate, and the extract evaporated to give crude 5-cyano-2-propoxybenzamidine, used directly in the next step.

f. A stirred suspension of ethyl 4-chloro-2-methylthiopyrimidine-4-carboxylate (1.91 g) in acetonitrile (20 ml) was cooled in an ice bath and a cold solution of the above crude amidine (1.67 g) in acetonitrile (20 ml) was added, followed by triethylamine (1.15 ml). After 2 hours in the cold the mixture was stirred at room temperature for 40 hours. After evaporation the residue was treated with water (30 ml) and extracted with dichloromethane (90 ml). The extract was evaporated and the residue purified by flash chromatography (silica gel, chloroform : methanol gradient) to give a solid. Recrystallisation yielded the title compound, 0.41 g, m.p. 226-228°C.

## EXAMPLE 2

### Preparation of 2-(5-carboxamido-2-propoxyphenyl)-7-methylthiopyrimido-[4,5-d]pyrimido-4(3H)-one

A stirred mixture of the product of Example 1 (0.41 g) and manganese dioxide on silica (4.1 g) in benzene (60 ml) was heated under reflux for 18 hours. After filtration the solid was washed with methanol containing 15% concentrated aqueous ammonia, the washings were evaporated, and the residue triturated with ether gave the title compound, 0.37 g, m.p. 259-261°C.

## EXAMPLE 3

### Preparation of 2-(5-carboxamido-2-propoxyphenyl)-7-cyclopropylamino-[4,5-d]pyrimido-4(3H)-one

a. A mixture of the product of Example 2 (0.37 g) and m-chloroperoxybenzoic acid (0.42 g) in dichloromethane (25 ml) was stirred at room temperature for 20 hours. Further m-chloroperoxybenzoic acid (0.2 g) and dichloromethane (15 ml) were added and the mixture stirred for 2 hours. After 3 days the mixture was chilled and filtered to give 2-(5-carboxamido-2-propoxyphenyl)-7-methylsulphonylpyrimido[4,5-d]-pyrimidin-4(3H)-one, 228 mg, m.p. 124-127°C. A further 166 mg was obtained from the organic solution, by evaporation, after washing it with sodium bicarbonate solution.

b. A solution of the above methylsulphonyl compound in dichloromethane is treated at room temperature

with an excess of cyclopropylamine to give the title compound.

EXAMPLE 4

Pharmaceutical compositions for oral administration are prepared by combining the following :

| | % w/w | | |
|---|---|---|---|
| 2-(5-carboxamido-2-propoxy phenyl)-7-cyclopropylamino-[4,5-d]pyrimido-4(3H)-one | 0.5 | 3.0 | 7.14 |
| 2% w/w Soya lecithin in soya bean oil | 90.45 | 88.2 | 84.41 |
| Hydrogenated vegetable shortening and beeswax | 9.05 | 8.8 | 8.45 |

The formulations are then filled into individual soft gelatin capsules.

EXAMPLE 5

A pharmaceutical composition for parenteral administration is prepared by dissolving the title compound of Example 3 (0.02 g) in polyethylene glycol 300 (25 ml) with heating. This solution is then diluted with water for injections Ph. Eur. (to 100 ml). The solution is then sterilised by filtration through a 0.22 micron membrane filter and sealed in sterile containers.

**Claims**

1. A compound of the formula (1) :

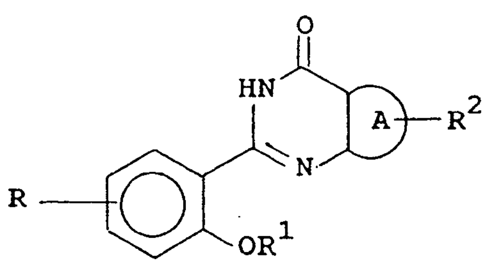

(1)

or a pharmaceutically acceptable salt thereof, wherein

$R^1$ is $C_{1-6}$alkyl, $C_{2-6}$alkenyl, $C_{3-5}$cycloalkyl $C_{1-6}$alkyl, or $C_{1-6}$alkyl substituted by 1 to 6 fluoro groups ;

$R^2$ is $C_{1-6}$alkylthio, $C_{1-6}$alkylsulphonyl, $C_{1-6}$alkoxy, hydroxy, hydrogen, hydrazino, $C_{1-6}$alkyl, phenyl, -$NHCOR^3$ wherein $R^3$ is hydrogen or $C_{1-6}$ alkyl, or -$NR^4R^5$, wherein $R^4$ and $R^5$ together with the nitrogen atom to which they are attached form a pyrrolidino, piperidino, hexahydroazepino, morpholino or piperazino ring, or $R^4$ and $R^5$ are independently hydrogen, $C_{3-5}$cycloalkyl or $C_{1-6}$alkyl which is optionally substituted by -$CF_3$, phenyl, -$S(O)_nC_{1-6}$ alkyl wherein

n is 0, 1 or 2, -$OR^6$, -$CO_2R^7$ or -$NR^8R^9$ wherein $R^6$ to $R^9$ are independently hydrogen or $C_{1-6}$alkyl, pro-

vided that the carbon atom adjacent to the nitrogen atom is not substituted by said $-S(O)_nC_{1-6}$alkyl, $-OR^6$ or $-NR^8R^9$ groups ; R is halo, $C_{1-4}$alkyl, $C_{1-4}$alkoxy, cyano, $-CONR^{10}R^{11}$, $CO_2R^{12}$, $C_{1-4}$ alkyl$S(O)_n$, $-NO_2$, $-NH_2$, $-NHCOR^{13}$ or $SO_2NR^{14}R^{15}$ wherein n is 0, 1 or 2 and $R^{10}$ to $R^{15}$ are independently hydrogen or $C_{1-4}$ alkyl ; and

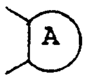 is a ring of sub-formula (a) or (b) :

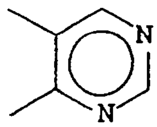

(a)

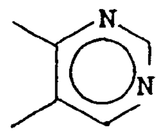

(b).

2. A compound according to claim 1 wherein $R^1$ is $C_{2-5}$alkyl.

3. A compound according to claim 1 wherein $R^1$ is n-propyl.

4. A compound according to any one of claims 1 to 3 wherein $R^2$ is $C_{1-6}$alkylthio, $C_{1-6}$alkylsulphonyl or $C_{1-6}$alkoxy.

5. A compound according to any one of claims 1 to 3 wherein $R^2$ is hydrogen, hydroxy or hydrazino.

6. A compound according to any one of claims 1 to 3 wherein $R^2$ is phenyl or $C_{1-6}$alkyl.

7. A compound according to any one of claims 1 to 3 wherein $R^2$ is $-NHCOR^3$ or $-NR^4R^5$.

8. A compound according to any one of claims 1 to 7 wherein R is halo, $C_{1-4}$alkyl or $C_{1-4}$alkoxy.

9. A compound according to any one of claims 1 to 7 wherein R is cyano, $-CONR^{10}R^{11}$ or $-CO_2R^{12}$ wherein $R^{12}$ is $C_{1-4}$alkyl.

10. A compound according to any one of claims 1 to 7 wherein R is $-NO_2$, $-NH_2$ or $-NHCOR^{13}$ wherein $R^{13}$ is $C_{1-4}$alkyl.

11. A compound according to any one of claims 1 to 7 wherein R is $C_{1-4}$alkyl$S(O)_n$ or $-SO_2NR^{14}R^{15}$.

12. A compound according to any one of claims 1 to 11

wherein 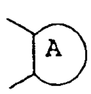 is a group of sub-formula (a).

13. A compound according to any one of claims 1 to 11

wherein 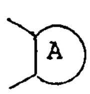 is a group of sub-formula (b).

14. A compound according to claim 1 which is :

2-(5-cyano-2-propoxyphenyl)-7-methylthiopyrimido-[4,5-d]]pyrimidin-4(3H)-one,
2-(5-carboxamido-2-propoxyphenyl)-7-methylthiopyrimido[4,5-d]pyrimido-4(3H)-one, or
2-(5-carboxamido-2-propoxyphenyl)-7-cyclopropylamino[4,5-d]pyrimido-4(3H)-one,

or a pharmaceutically acceptable salt thereof.

**15.** A compound according to any one of claims 1 to 14 for use as a medicament.

**16.** A pharmaceutical composition which comprises a compound according to any one of claims 1 to 14 and a pharmaceutically acceptable carrier.

**17.** A process for preparing a compound of the formula (1) or a pharmaceutically acceptable salt thereof as defined in claim 1 which comprises :

a) cyclising a compound of the formula (2) :

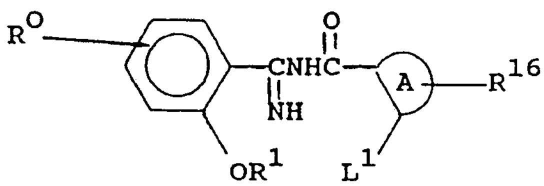

(2)

wherein $L^1$ is a displaceable group, $R^1$ and 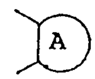 are as defined in claim 1, $R^{16}$ is a group $R^2$ as defined in claim 1 or a precursor thereof, and $R^0$ is a group R as defined in claim 1 or a precursor thereof ; or

b) cyclising a compound of the formula (3) :

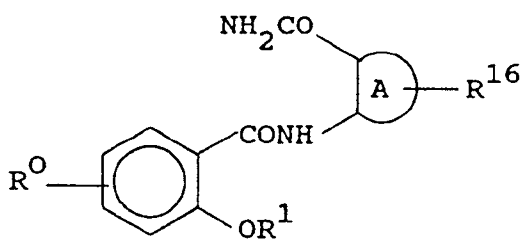

(3)

wherein $R^0$, $R^1$, $R^{16}$ and 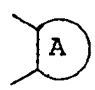 are as hereinbefore defined;

and thereafter where necessary :

. converting a group $R^{16}$ to a group $R^2$ ;
. converting a group $R^0$ to a group R ;
. optionally forming a pharmaceutically acceptable salt.

**18.** A compound of the formula (2) as defined in claim 17.

**19.** A compound of the formula (3) as defined in claim 17.